# FASCICULE DE BREVET EUROPEEN

(11) **EP 1 488 783 B1**
(45) Date de publication et mention de la délivrance du brevet: **22.11.2006**
(21) Numéro de dépôt: 04291517.3
(22) Date de dépôt: 16.06.2004
(51) Int. Cl.: A61K 8/49, A61Q 5/10

(54) **Composition tinctoriale comprenant le 4,5-diamino-1-(bêta-hydroxyéthyl)-1H-pyrazole ou le 4,5-diamino-1-(bêta-méthoxyéthyl)-1H-pyrazole à titre de base d'oxydation et la 2,6-dihydroxy-3,4-diméthyl pyridine à titre de coupleur**
Färbemittel enthaltend 4,5-Diamino-1-(beta-Hydroxyethyl)-1H-Pyrazol oder 4,5-Diamino-1-(beta-Methoxyethyl)-1H-Pyrazol als Entwickler und 2,6-Dihydroxy-3,4-Dimethylpyridin als Kuppler
Dyeing composition comprising 4,5-diamino-1-(beta-hydroxyethyl)-1H-pyrazole or 4,5-diamino-1-(beta-methoxyethyl)-1H-pyrazole as oxidation base and 2,6-dihydroxy-3,4-dimethylpyridine as coupling agent

(30) Priorité: 19.06.2003 FR 0307400
(43) Date de publication de la demande: 22.12.2004
(73) Titulaire: L'ORÉAL, 75008 Paris (FR)
(72) Inventeur: Audousset, Marie-Pascale, 92600 Asnières (FR)
(74) Mandataire: Fevrier, Murielle Françoise E.

(56) Documents cités:
- WO-A-02/46165
- DE-A- 19 962 871
- DE-U- 20 118 089
- US-A- 5 785 717

## Description

L'invention a pour objet une composition pour la teinture d'oxydation des fibres kératiniques, et en particulier des fibres kératiniques humaines telles que les cheveux, comprenant le 4,5-diamino-1-(β-hydroxyéthyl)-1H-pyrazole ou le 4,5-diamino-1-(β-méthoxyéthyl)-1H-pyrazole à titre de base d'oxydation et la 2,6-dihydroxy-3,4-diméthyl pyridine à titre de coupleur.

Il est connu de teindre les fibres kératiniques, et en particulier les fibres kératiniques humaines telles que les cheveux, avec des compositions tinctoriales contenant des précurseurs de colorant d'oxydation, appelés généralement bases d'oxydation, tels que des ortho- ou para-phénylènediamines, des ortho- ou para-aminophénols et des composés hétérocycliques. Ces bases d'oxydation sont des composés incolores ou faiblement colorés qui, associés à des produits oxydants, peuvent donner naissance par un processus de condensation oxydative à des composés colorés.

On sait également que l'on peut faire varier les nuances obtenues avec ces bases d'oxydation en les associant à des coupleurs ou modificateurs de coloration, ces derniers étant choisis notamment parmi les méta-diamines aromatiques, les méta-aminophénols, les méta-diphénols et certains composés hétérocycliques tels que des composés indoliques.

La variété des molécules mises en jeu au niveau des bases d'oxydation et des coupleurs permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs tels que la lumière, les intempéries, le lavage, les ondulations permanentes, la transpiration et les frottements.

Les colorants doivent également permettre de couvrir les cheveux blancs, et être enfin les moins sélectifs possibles, c'est-à-dire permettre d'obtenir des écarts de coloration les plus faibles possibles tout au long d'une même fibre kératinique, qui est en général différemment sensibilisée (i.e. abîmée) entre sa pointe et sa racine.

Il a déjà été proposé, notamment dans la demande de brevet EP 0 375 977, des compositions pour la coloration d'oxydation des fibres kératiniques comprenant un dérivé de 3,4- ou 4,5-diamino pyrazole à titre de base d'oxydation, en association avec des coupleurs classiquement utilisés pour la coloration d'oxydation des fibres kératiniques.

Le brevet USA-5 785 717 décrit des compositions pour la teinture des fibres kératiniques comprenant an moins un dérivé diaminopyrazole comme base d'oxydation et au moins un dérivé hétérocyclique comme coupleur.

Les demandes de brevet allemand DE 199 62 871 et DE 199 62 872 décrivent des compositions pour la teinture d'oxydation des fibres kératiniques comprenant un dérivé de 4,5-diamino pyrazole à titre de base d'oxydation, en association avec un dérivé de méta-aminophénol convenablement sélectionné à titre de coupleur.

La demande de brevet allemand DE 100 37 158 décrit des compositions pour la teinture d'oxydation des fibres kératiniques comprenant un dérivé de 4,5-diamino pyrazole et un dérivé de la para-phénylènediamine convenablement sélectionnés à titre de bases d'oxydation, en association avec des coupleurs classiquement utilisés en coloration d'oxydation des fibres kératiniques.

La demande de brevet allemand DE 201 18 089 décrit des compositions pour la teinture d'oxydation des fibres kératiniques comprenant un dérivé de 4,5-diamino pyrazole de formule générale déterminée à titre de base d'oxydation, en association avec des coupleurs classiquement utilisés en coloration d'oxydation des fibres kératiniques.

La demande de brevet WO 02/46165 décrit des compositions pour la teinture d'oxydation des fibres kératiniques comprenant un dérivé de 4,5-diamino pyrazole de formule générale déterminée à titre de base d'oxydation.

La demande de brevet WO 03/028688 décrit des compositions pour la teinture d'oxydation des fibres kératiniques comprenant un dérivé de 4,5-diamino pyrazole de formule générale déterminée à titre de base d'oxydation, en association avec des bases d'oxydation hétérocycliques autres que les dérivés de 4,5-diamino pyrazole et des coupleurs classiquement utilisés en coloration d'oxydation des fibres kératiniques.

Par ailleurs, la demande de brevet allemand DE 199 27 074 décrit des compositions pour la coloration d'oxydation des fibres kératiniques comprenant un coupleur convenablement sélectionné, en association avec un dérivé de la pyridine de formule générale déterminée à titre de base d'oxydation.

Les compositions décrites dans l'art antérieur ne sont cependant pas entièrement satisfaisantes notamment au niveau de la variété des nuances qui peuvent être obtenues ainsi que du point de vue de la tenue des colorations obtenues vis-à-vis des diverses agressions que peuvent subir les cheveux, et en particulier vis-à-vis des shampooings et des déformations permanentes et du point de vue de la puissance des colorations obtenues.

Le but de la présente invention est de fournir de nouvelles compositions pour la teinture d'oxydation des fibres kératiniques ne présentant pas les inconvénients de celles de l'art antérieur. En particulier, le but de la présente invention est de fournir de nouvelles compositions conduisant à des colorations originales, puissantes, esthétiques, peu sélectives et résistant bien aux diverses agressions que peuvent subir les cheveux telles que les shampooings, la lumière, la sueur et les déformations permanentes.

Ce but est atteint avec la présente invention qui a pour objet une composition pour la teinture d'oxydation des fibres kératiniques comprenant, dans un milieu de teinture approprié :
- au moins une base d'oxydation choisie parmi le 4,5-diamino-1-(β-hydroxyéthyl)-1H-pyrazole, le 4,5-diamino-1-(β-méthoxyéthyl)-1H-pyrazole et ses sels d'addition ; et
- au moins un coupleur choisi parmi la 2,6-dihydroxy-3,4-diméthyl pyridine et ses sels d'addition.

La composition de la présente invention permet en particulier d'obtenir une coloration des fibres kératiniques dans des nuances plus chaudes telles que des nuances cuivrées, puissante, esthétique et peu sélective.

L'invention a aussi pour objet un procédé de teinture d'oxydation des fibres kératiniques, et en particulier des fibres kératiniques humaines telles que les cheveux, mettant en oeuvre cette composition.

Un autre objet de l'invention est l'utilisation de la composition de la présente invention pour la teinture d'oxydation des fibres kératiniques, et en particulier des fibres kératiniques humaines telles que les cheveux.

La composition de la présente invention peut en outre comprendre une ou plusieurs bases d'oxydation additionnelles choisies parmi les bases d'oxydation classiquement utilisées en teinture d'oxydation différentes du 4,5-diamino-1-(β-hydroxyéthyl)-1H-pyrazole et du 4,5-diamino-1-(β-méthoxyéthyl)-1H-pyrazole. A titre d'exemple, ces bases d'oxydation additionnelles sont choisies parmi les para-phénylènediamines, les bis-phénylalkylènediamines, les para-aminophénols, les ortho-aminophénols, les bases hétérocycliques autres que le 4,5-diamino-1-(β-hydroxyéthyl)-1H-pyrazole et le 4,5-diamino-1-(β-méthoxyéthyl)-1 H-pyrazole et leurs sels d'addition.

Parmi les para-phénylènediamines, on peut citer à titre d'exemple, la para-phénylènediamine ; la para-toluylènediamine ; la2-chloro para-phénylènediamine ; la 2,3-diméthyl para-phénylènediamine ; la 2,6-diméthyl para-phénylènediamine ; la 2,6-diéthyl para-phénylènediamine ; la 2,5-diméthyl para-phénylènediamine ; la N,N-diméthyl para-phénylènediamine ; la N,N-diéthyl para-phénylènediamine ; la N,N-dipropyl para-phénylènediamine ; la 4-amino N,N-diéthyl 3-méthyl aniline ; la N,N-bis-(β-hydroxyéthyl) para-phénylènediamine ; la 4-N,N-bis-(β-hydroxyéthyl)amino 2-méthyl aniline ; la 4-N,N-bis-(fi-hydroxyéthyl)amino 2-chloro aniline ; la 2-(β-hydroxyéthyt) para-phénylènediamine ; la 2-fluoro para-phénylènediamine ; la 2-isopropyl para-phénylènediamine ; la N-(β-hydroxypropyl) para-phénylènediamine ; la 2-hydroxyméthyl para-phénylènediamine ; la N,N-diméthyl 3-méthyl para-phénylènediamine ; la N,N-(éthyl, β-hydroxyéthyl) para-phénylènediamine ; la N-(β,γ-dihydroxypropyl) para-phénylènediamine ; la N-(4'-aminophényl) para-phénylènediamine ; la N-phényl para-phénylènediamine ; la 2-(β-hydroxyéthyloxy) para-phénylènediamine ; la 2-(β-acétylaminoéthyloxy) para-phénylènediamine ; la N-(β-méthoxyéthyl) para-phénylènediamine ; la 2-thiényl para-phénylènediamine ; le 2-(β-hydroxyéthylamino) 5-amino toluène et leurs sels d'addition.

Parmi les para-phénylènediamines citées ci-dessus, la para-phénylènediamine ; la para-toluylènediamine ; la 2-isopropyl para-phénylènediamine ; la 2-(β-hydroxyéthyl) para-phénylènediamine ; la 2-(β-hydroxyéthyloxy) para-phénylènediamine ; la 2,6-diméthyl para-phénylènediamine ; la 2,6-diéthyl para-phénylènediamine ; la 2,3-diméthyl para-phénylènediamine ; la N,N-bis-(β-hydroxyéthyl) para-phénylènediamine ; la 2-chloro para-phénylènediamine ; la 2-(β-acétylaminoéthyloxy) para-phénylènediamine et leurs sels d'addition sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol ; la N,N'-bis-(β-hydroxyéthyl) N,N'-bis-(4'-aminophényl) éthylènediamine ; la N,N'-bis-(4-aminophényl) tétraméthylènediamine ; la N,N'-bis-(β-hydroxyéthyl) N,N'-bis-(4-aminophényl) tétraméthylènediamine ; la N,N'-bis-(4-méthylaminophényl) tétraméthylènediamine ; la N,N'-bis-(éthyl) N,N'-bis-(4'-amino, 3'-méthylphényl) éthylènediamine ; le 1,8-bis-(2,5-diamino phénoxy)-3,6-dioxaoctane et leurs sels d'addition.

Parmi les para-aminophénols, on peut citer à titre d'exemple, le para-aminophénol ; le 3-méthyl 4-amino phénol ; le 4-amino 3-fluoro phénol ; le 4-amino 3-hydroxyméthyl phénol ; le 4-amino 2-méthyl phénol ; le 4-amino 2-hydroxyméthyl phénol ; le 4-amino 2-méthoxyméthyl phénol ; le 4-amino 2-aminométhyl phénol ; le 4-amino 2-(β-hydroxyéthyl aminométhyl) phénol ; le 4-amino 2-fluoro phénol ; le 4-amino 2-chloro phénol ; le 4-amino 2,6-dichloro phénol ; le 4-amino 6-((5'-amino 2'-hydroxy 3'-méthyl phényl)méthyl) 2-méthyl phénol ; le bis-(5-amino 2-hydroxy phényl) méthane et leurs sels d'addition.

Parmi les ortho-aminophénols, on peut citer à titre d'exemple, le 2-amino phénol ; le 2-amino 5-méthyl phénol ; le 2-amino 6-méthyl phénol ; le 5-acétamido 2-amino phénol et leurs sels d'addition.

Parmi les bases hétérocycliques, on peut citer à titre d'exemple, les dérivés pyridiniques, les dérivés pyrimidiniques et les dérivés pyrazoliques autres que le 4,5-diamino-1-(β-hydroxyéthyl)-1H-pyrazole et le 4,5-diamino-1-(β-méthoxyéthyl)-1H-pyrazole.

Parmi les dérivés pyridiniques, on peut citer les composés décrits par exemple dans les brevets GB 1 026 978 et GB 1 153 196, comme la 2,5-diamino pyridine ; la 2-(4-méthoxyphényl)amino 3-amino pyridine ; la 2,3-diamino 6-méthoxy pyridine ; la 2-(β-méthoxyéthyl)amino 3-amino 6-méthoxy pyridine ; la 3,4-diamino pyridine et leurs sels d'addition.

D'autres bases d'oxydation pyridiniques utiles dans la présente invention sont les bases d'oxydation 3-amino pyrazolo-[1,5-a]-pyridines ou leurs sels d'addition décrits par exemple dans la demande de brevet FR 2 801 308. A titre d'exemple, on peut citer la pyrazolo[1,5-a]pyridin-3-ylamine ; la 2-acétylamino pyrazolo-[1,5-a] pyridin-3-ylamine ; la 2-morpholin-4-yl-pyrazolo[1,5-a]pyridin-3-ylamine ; l'acide 3-amino-pyrazolo[1,5-a]pyridin-2-carboxylique ; la 2-méthoxy-pyrazolo[1,5-a]pyridine-3-ylamino ; le (3-amino-pyrazolo[1,5-a]pyridine-7-yl)-méthanol ; le 2-(3-amino-pyrazolo[1,5-a]pyridine-5-yl)-éthanol ; le 2-(3-amino-pyrazolo[1,5-a]pyridine-7-yl)-éthanol ; le (3-amino-pyrazolo[1,5-a]pyridine-2-yl)-méthanol ; la 3,6-diamino-pyrazolo[1,5-a]pyridine ; la 3,4-diamino-pyrazolo[1,5-a]pyridine ; la pyrazolo[1,5-a]pyridine-3,7-diamine ; la 7-morpholin-4-yl-pyrazolo[1,5-a]pyridin-3-ylamine ; la pyrazolo[1,5-a]pyridine-3,5-diamine ; la 5-morpholin-4-yl-pyrazolo[1,5-a]pyridin-3-ylamine ; le 2-[(3-amino-pyrazolo[1,5-a]pyridin-5-yl)-(2-hydroxyéthyl)-amino]-éthanol ; le 2-[(3-amino-pyrazolo[1,5-a]pyridin-7-yl)-(2-hydroxyéthyl)-amino]-éthanol ; la 3-amino-pyrazolo[1,5-a]pyridine-5-ol ; 3-amino-pyrazolo[1,5-a]pyridine-4-ol ; la 3-amino-pyrazolo[1,5-a]pyridine-6-ol ; la 3-amino-pyrazolo[1,5-a]pyridine-7-ol ; ainsi que leurs sels d'addition.

Parmi les dérivés pyrimidiniques, on peut citer les composés décrits par exemple dans les brevets DE 23 59 399 ; JP 88-169571 ; JP 05-63124 ; EP 0 770 375 ou demande de brevet WO 96/15765 comme la 2,4,5,6-tétra-aminopyrimidine ; la 4-hydroxy 2,5,6-triaminopyrimidine ; la 2-hydroxy 4,5,6-triaminopyrimidine ; la 2,4-dihydroxy 5,6-diaminopyrimidine ; la 2,5,6-triaminopyrimidine et leurs sels d'addition et leurs formes tautomères, lorsqu'il existe un équilibre tautomérique.

Parmi les dérivés pyrazoliques autres que le 4,5-diamino-1-(β-hydroxyéthyl)-1 H-pyrazole et le 4,5-diamino-1-(β-méthoxyéthyl)-1H-pyrazole, on peut citer les composés décrits dans les brevets DE 38 43 892, DE 41 33 957 et demandes de brevet WO 94/08969, WO 94/08970, FR-A-2 733 749, DE 195 43 988 et WO 02/46165 comme le 4,5-diamino 1-méthyl pyrazole ; le 3,4-diamino pyrazole ; le 4,5-diamino 1-(4'-chlorobenzyl) pyrazole ; le 4,5-diamino 1,3-diméthyl pyrazole ; le 4,5-diamino 3-méthyl 1-phényl pyrazole ; le 4,5-diamino 1-méthyl 3-phényl pyrazole ; le 4-amino 1,3-diméthyl 5-hydrazino pyrazole ; le 1-benzyl 4,5-diamino 3-méthyl pyrazole ; le 4,5-diamino 3-*tert*-butyl 1-méthyl pyrazole ; le 4,5-diamino 1-*tert*-butyl 3-méthyl pyrazole ; le 4,5-diamino 1-éthyl 3-méthyl pyrazole ; le 4,5-diamino 1-éthyl 3-(4'-méthoxyphényl) pyrazole ; le 4,5-diamino 1-éthyl 3-hydroxyméthyl pyrazole ; le 4,5-diamino 3-hydroxyméthyl 1-méthyl pyrazole ; le 4,5-diamino 3-hydroxyméthyl 1-isopropyl pyrazole ; le 4,5-diamino 3-méthyl 1-isopropyl pyrazole ; le 4-amino 5-(2'-aminoéthyl)amino 1,3-diméthyl pyrazole ; le 3,4,5-triamino pyrazole ; le 1-méthyl 3,4,5-triamino pyrazole ; le 3,5-diamino 1-méthyl 4-méthylamino pyrazole ; le 3,5-diamino 4-(β-hydroxyéthyl)amino 1-méthyl pyrazole et leurs sels d'addition.

En présence de bases d'oxydation additionnelles, celles-ci sont de préférence choisies parmi les bis-phénylalkylènediamines, les para-aminophénols, les ortho-aminophénols, les bases hétérocycliques autres que le 4,5-diamino-1-(β-hydroxyéthyl)-1H-pyrazole et le 4,5-diamino-1-(β-méthoxyéthyl)-1H-pyrazole et leurs sels d'addition.

De préférence, la composition de l'invention comprend comme unique base d'oxydation au moins une base d'oxydation choisie parmi le 4,5-diamino-1-(β-hydroxyéthyl)-1H-pyrazole, le 4,5-diamino-1-(β-méthoxyéthyl)-1H-pyrazole et leurs sels d'addition.

Les bases d'oxydation présentes dans la composition de l'invention sont en général présentes chacune en quantité comprise entre 0,001 et 10 % en poids environ du poids total de la composition tinctoriale, de préférence entre 0,005 et 6 %.

La composition selon l'invention peut contenir en plus de la 2,6-dihydroxy-3,4-diméthyl pyridine un ou plusieurs coupleurs additionnels choisis parmi les coupleurs conventionnellement utilisés pour la teinture d'oxydation des fibres kératiniques. Parmi ces coupleurs, on peut notamment citer les méta-phénylènediamines, les méta-aminophénols, les méta-diphénols, les coupleurs naphtaléniques, les coupleurs hétérocycliques autres que la 2,6-dihydroxy-3,4-diméthyl pyridine ainsi que leur sels d'addition.

A titre d'exemple, on peut citer le 2-méthyl 5-amino phénol ; le 5-N-(β-hydroxyéthyl)amino 2-méthyl phénol ; le 6-chloro-2-méthyl-5-amino phénol ; le 3-amino phénol ; le 1,3-dihydroxy benzène ; le 1,3-dihydroxy 2-méthyl benzène ; le 4-chloro 1,3-dihydroxy benzène ; le 2,4-diamino 1-(β-hydroxyéthyloxy) benzène ; le 2-amino 4-(β-hydroxyéthylamino) 1-méthoxy benzène ; le 1,3-diamino benzène ; le 1,3-bis-(2,4-diaminophénoxy) propane ; la 3-uréido aniline ; le 3-uréido 1-diméthylamino benzène ; le sésamol ; le 1-β-hydroxyéthylamino-3,4-méthylènedioxy benzène ; l'α-naphtol ; le 2 méthyl-1-naphtol ; le 6-hydroxy indole ; le 4-hydroxy indole ; le 4-hydroxy N-méthyl indole ; la 2-amino-3-hydroxy pyridine ; la 6- hydroxy benzomorpholine ; la 3,5-diamino-2,6-diméthoxy pyridine ; le 1-N-(β-hydroxyéthyl)amino-3,4-méthylènedioxy benzène ; le 2,6-bis-(β-hydroxyéthylamino) toluène et leurs sels d'addition.

Selon un mode de réalisation particulier de l'invention, la composition comprend comme unique coupleur au moins un coupleur choisi parmi la 2,6-dihydroxy 3,4-diméthyl pyridine et ses sels d'addition.

Dans la composition de la présente invention, le ou les coupleurs sont chacun généralement présents en quantité comprise entre 0,001 et 10 % en poids environ du poids total de la composition tinctoriale, de préférence entre 0,005 et 6 %.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates et les sels d'addition avec une base telles que la soude, la potasse, l'ammoniaque, les amines ou les alcanolamines.

La composition tinctoriale conforme à l'invention peut en outre comprendre un ou plusieurs colorants directs pouvant notamment être choisis parmi les colorants nitrés de la série benzénique, les colorants directs azoïques, les colorants directs méthiniques. Ces colorants directs peuvent être de nature non ionique, anionique ou cationique.

Le milieu approprié pour la teinture appelé aussi support de teinture est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants peuvent être présents dans des proportions comprises entre 1 et 40 % en poids environ par rapport au poids total de la composition tinctoriale, et encore plus préférentiellement entre 5 et 30 % en poids environ.

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Les adjuvants ci-dessus peuvent être présents en quantité comprise pour chacun d'eux entre 0,01 et 20 % en poids par rapport au poids de la composition.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

Le pH de la composition tinctoriale conforme à l'invention est généralement compris entre 3 et 12 environ, de préférence entre 7 et 12 environ, et encore plus préférentiellement entre 7,5 et 11 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (I) suivante : 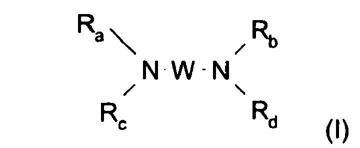 dans laquelle W est un reste propylène éventuellement substitué par un groupement hydroxyle ou un radical alkyle en C₁-C₄ ; Rₐ, R_{b}, R_{c} et R_{d}, identiques ou différents, représentent un atome d'hydrogène, un radical alkyle en C₁-C₄ ou hydroxyalkyle en C₁-C₄.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

Le procédé de la présente invention est un procédé dans lequel la composition conforme à l'invention telle que définie précédemment est appliquée sur les fibres kératiniques, et la couleur est révélée à l'aide d'un agent oxydant. La couleur peut être révélée à pH acide, neutre ou alcalin. De préférence, la couleur est révélée à pH compris entre 7 et 12. L'agent oxydant peut être ajouté à la composition de l'invention juste au moment de l'emploi ou il peut être mis en oeuvre à partir d'une composition oxydante le contenant, appliquée simultanément ou séquentiellement à la composition de l'invention.

Selon un mode de réalisation particulier, la composition selon la présente invention est mélangée, de préférence au moment de l'emploi, à une composition contenant, dans un milieu approprié pour la teinture, au moins un agent oxydant, cet agent oxydant étant présent en une quantité suffisante pour développer une coloration. Le mélange obtenu est ensuite appliqué sur les fibres kératiniques. Après un temps de pose de 3 à 50 minutes environ, de préférence 5 à 30 minutes environ, les fibres kératiniques sont rincées, lavées au shampooing, rincées à nouveau puis séchées.

Les agents oxydants classiquement utilisés pour la teinture d'oxydation des fibres kératiniques sont par exemple le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydo-réductases à 2 électrons telles que les uricases et les oxygénases à 4 électrons comme les laccases. Le peroxyde d'hydrogène est particulièrement préféré.

La composition oxydante peut également renfermer divers adjuvants utilisés classiquement dans les compositions pour la teinture des cheveux et tels que définis précédemment.

Le pH de la composition oxydante renfermant l'agent oxydant est tel qu'après mélange avec la composition tinctoriale, le pH de la composition résultante appliquée sur les fibres kératiniques varie de préférence entre 3 et 12 environ, de préférence entre 7 et 12 environ, et encore plus préférentiellement entre 7,5 et 11 environ. Il peut être ajusté à la valeur désirée au moyen d'agents acidifiants ou alcalinisants habituellement utilisés en teinture des fibres kératiniques et tels que définis précédemment.

La composition prête à l'emploi qui est finalement appliquée sur les fibres kératiniques peut se présenter sous des formes diverses, telles que sous forme de liquides, de crèmes, de gels ou sous toute autre forme appropriée pour réaliser une teinture des fibres kératiniques, et notamment des cheveux humains.

L'invention a aussi pour objet un dispositif à plusieurs compartiments ou "kit" de teinture dans lequel un premier compartiment renferme la composition tinctoriale conforme à l'invention et un deuxième compartiment renferme une composition oxydante. Ce dispositif peut être équipé d'un moyen permettant de délivrer sur les cheveux le mélange souhaité, tel que les dispositifs décrits dans le brevet FR-2 586 913 au nom de la demanderesse.

A partir de ce dispositif, il est possible de teindre les fibres kératiniques à partir d'un procédé qui comprend le mélange d'une composition tinctoriale conforme à l'invention avec un agent oxydant tel que défini précédemment, et l'application du mélange obtenu sur les fibres kératiniques pendant un temps suffisant pour développer la coloration désirée.

Les exemples qui suivent servent à illustrer l'invention sans toutefois présenter un caractère limitatif.

### EXEMPLES

Une composition tinctoriale est réalisée comme indiqué ci-dessous :

| **Exemple** | **1** |
|---|---|
| 4,5-diamino-1-(β-hydroxyéthyl)-1H-pyrazole, 2 HCl | 1,075 g |
| 2,6-dihydroxy-3,4-diméthyl pyridine | 0,695 g |
| Support de teinture | (*) |
| Eau déminéralisée q.s.p. | 100 g |

| | |
|---|---|
| (*) : support de teinture commun | |

| | |
|---|---|
| Alcool oléique polyglycérolé à 2 moles de glycérol | 4 g |
| Alcool oléique polyglycérolé à 4 moles de glycérol (78 % M.A.) | 5,69 g M.A. |
| Acide oléique | 3,0 g |
| Amine oléique à 2 moles d'oxyde d'éthylène vendue sous la dénomination commerciale ETHOMEEN O12 par la société AKZO | 7 g |
| Laurylamino succinamate de diéthylaminopropyle, sel de sodium à 55% M.A. | 3,0 g M.A. |
| Alcool oléique | 5,0 g |
| Diéthanolamide d'acide oléique | 12,0 g |
| Propylène glycol | 3,5 g |
| Alcool éthylique | 7,0 g |
| Dipropylène glycol | 0,5 g |
| Monométhyléther de propylène glycol | 9,0 g |
| Métabisulfite de sodium en solution aqueuse à 35% M.A. | 0,455 g M.A. |
| Acétate d'ammonium | 0,8 g |
| Antioxydant, séquestrant | q.s. |
| Parfum, conservateur | q.s. |
| Ammoniaque à 20% de NH₃ | 10,0 g |

La composition est mélangée extemporanément avec 1 fois son volume d'eau oxygénée à 20 volumes, et dont le pH est 3.
Le pH de la composition obtenue après mélange est de l'ordre de 9,8.
Le mélange ainsi réalisé est appliqué sur des mèches de cheveux gris à 90% de blancs permanentés, à raison de 30 g pour 3 g de cheveux, pendant 30 minutes. Les cheveux sont ensuite rincés, lavés avec un shampooing standard et séchés.
La coloration capillaire est évaluée de manière visuelle. Les nuances obtenues figurent dans le tableau ci-dessous.

| | |
|---|---|
| Hauteur de ton | reflet |
| Châtain foncé | Cuivré doré intense |

## Revendications

1. Composition pour la teinture d'oxydation des fibres kératiniques comprenant, dans un milieu de teinture approprié :
- au moins une base d'oxydation choisie parmi le 4,5-diamino-1-(β-hydroxyéthyl)-1H-pyrazole, le 4,5-diamino-1-(β-méthoxyéthyl)-1H-pyrazole et ses sels d'addition ; et
- au moins un coupleur choisi parmi la 2,6-dihydroxy-3,4-diméthyl pyridine et ses sels d'addition.

2. Composition selon la revendication 1, comprenant au moins une base d'oxydation additionnelle choisie parmi les para-phénylènediamines, les bis-phénylalkylènediamines, les para-aminophénols, les ortho-aminophénols, les bases hétérocycliques autres que le 4,5-diamino-1-(β-hydroxyéthyl)-1H-pyrazole et le 4,5-diamino-1-(β-méthoxyéthyl)-1H-pyrazole ainsi que leurs sels d'addition.

3. Composition selon la revendication 2, dans laquelle les bases d'oxydation sont choisies parmi les bis-phénylalkylènediamines, les para-aminophénols, les ortho-aminophénols, les bases hétérocycliques autres que le 4,5-diamino-1-(β-hydroxyéthyl)-1H-pyrazole et le 4,5-diamino-1-(β-méthoxyéthyl)-1 H-pyrazole ainsi que leurs sels d'addition.

4. Composition selon la revendication 1, comprenant comme unique base d'oxydation au moins une base d'oxydation choisie parmi le 4,5-diamino-1-(β-hydroxyéthyl)-1H-pyrazole, le 4,S-diamino-1-(β-méthoxyéthyl)-1H-pyrazole et leurs sels d'addition.

5. Composition selon l'une quelconque des revendications précédentes, comprenant au moins un coupleur additionnel choisi parmi les méta-phénylènediamines, les méta-aminophénols, les méta-diphénols, les coupleurs naphtaléniques, les coupleurs hétérocycliques autres que la 2,6-dihydroxy-3,4-diméthyl pyridine ainsi que leurs sels d'addition.

6. Composition selon l'une quelconque des revendications précédentes, dans laquelle la quantité de chacune des bases d'oxydation est comprise entre 0,001 et 10 % en poids environ du poids total de la composition tinctoriale.

7. Composition selon l'une quelconque des revendications précédentes, dans laquelle la quantité de chacun des coupleurs est comprise entre 0,001 et 10 % en poids environ du poids total de la composition tinctoriale.

8. Composition selon l'une quelconque des revendications précédentes, ayant un pH compris entre 3 et 12.

9. Composition selon la revendication 8, ayant un pH compris entre 7 et 12.

10. Composition selon l'une quelconque des revendications précédentes, comprenant un agent oxydant.

11. Composition selon la revendication 10, dans laquelle l'agent oxydant est choisi parmi le peroxyde d'hydrogène, le peroxyde d'urée, les bromates de métaux alcalins, les persels, les peracides et les enzymes oxydases.

12. Composition selon la revendication 10 ou 11, ayant un pH compris entre 7 et 12.

13. Procédé de teinture d'oxydation des fibres kératiniques, **caractérisé en ce qu'**une composition telle que définie à l'une quelconque des revendications 1 à 9 est appliquée sur les fibres kératiniques, et que la couleur est révélée à l'aide d'un agent oxydant.

14. Procédé selon la revendication 13, dans lequel la couleur est révélée à pH compris entre 7 et 12.

15. Procédé selon la revendication 13 ou 14, dans lequel l'agent oxydant est choisi parmi le peroxyde d'hydrogène, le peroxyde d'urée, les bromates de métaux alcalins, les persels, les peracides et les enzymes oxydases.

16. Procédé selon l'une quelconque des revendications 13 à 15, dans lequel l'agent oxydant est mélangé au moment de l'emploi à la composition telle que définie à l'une quelconque des revendications 1 à 9.

17. Procédé selon l'une quelconque des revendications 13 à 15, dans lequel l'agent oxydant est appliqué sur les fibres kératiniques sous forme d'une composition oxydante simultanément ou séquentiellement à la composition telle que définie à l'une quelconque des revendications 1 à 9.

18. Dispositif à plusieurs compartiments, dans lequel un premier compartiment contient une composition tinctoriale telle que définie à l'une quelconque des revendications 1 à 9 et un deuxième compartiment contient un agent oxydant.

19. Utilisation de la composition tinctoriale telle que définie à l'une quelconque des revendications 1 à 12 pour la teinture d'oxydation des fibres kératiniques.

## Claims

1. Composition for the oxidation dyeing of keratin fibres, comprising, in a suitable dyeing medium:
- at least one oxidation base chosen from 4,5-diamino-1-(β-hydroxyethyl)-1H-pyrazole and 4,5-diamino-1-(β-methoxyethyl)-1H-pyrazole, and the addition salts thereof; and
- at least one coupler chosen from 2,6-dihydroxy-3,4-dimethylpyridine and the addition salts thereof.

2. Composition according to Claim 1, comprising at least one additional oxidation base chosen from paraphenylenediamines, bis(phenyl)alkylenediamines, para-aminophenols, ortho-aminophenols and heterocyclic bases other than 4,5-diamino-1-(β-hydroxyethyl)-1H-pyrazole and 4,5-diamino-1-(β-methoxyethyl)-1H-pyrazole, and the addition salts thereof.

3. Composition according to Claim 2, in which the oxidation bases are chosen from bis(phenyl)alkylenediamines, para-aminophenols, ortho-aminophenols and heterocyclic bases other than 4,5-diamino-1-(β-hydroxyethyl)-1H-pyrazole and 4,5-diamino-1-(β-methoxyethyl)-1H-pyrazole, and the addition salts thereof.

4. Composition according to Claim 1, comprising as sole oxidation base at least one oxidation base chosen from 4,5-diamino-1-(β-hydroxyethyl)-1H-pyrazole and 4,5-diamino-1-(β-methoxyethyl)-1H-pyrazole, and the addition salts thereof.

5. Composition according to any one of the preceding claims, comprising at least one additional coupler chosen from meta-phenylenediamines, meta-aminophenols, meta-diphenols, naphthalene-based couplers and heterocyclic couplers other than 2,6-dihydroxy-3,4-dimethylpyridine, and the addition salts thereof.

6. Composition according to any one of the preceding claims, in which the amount of each of the oxidation bases is between 0.001% and 10% by weight approximately relative to the total weight of the dye composition.

7. Composition according to any one of the preceding claims, in which the amount of each of the couplers is between 0.001% and 10% by weight approximately relative to the total weight of the dye composition.

8. Composition according to any one of the preceding claims, having a pH of between 3 and 12.

9. Composition according to Claim 8, having a pH of between 7 and 12.

10. Composition according to any one of the preceding claims, comprising an oxidizing agent.

11. Composition according to Claim 10, in which the oxidizing agent is chosen from hydrogen peroxide, urea peroxide, alkali metal bromates, persalts, peracids and oxidase enzymes.

12. Composition according to Claim 10 or 11, having a pH of between 7 and 12.

13. Process for the oxidation dyeing of keratin fibres, **characterized in that** a composition as defined in any one of Claims 1 to 9 is applied to the keratin fibres, and the colour is developed using an oxidizing agent.

14. Process according to Claim 13, in which the colour is developed at a pH of between 7 and 12.

15. Process according to Claim 13 or 14, in which the oxidizing agent is chosen from hydrogen peroxide, urea peroxide, alkali metal bromates, persalts, peracids and oxidase enzymes.

16. Process according to any one of Claims 13 to 15, in which the oxidizing agent is mixed at the time of use with the composition as defined in any one of Claims 1 to 9.

17. Process according to any one of Claims 13 to 15, in which the oxidizing agent is applied to the keratin fibres in the form of an oxidizing composition simultaneously with or sequentially to the composition as defined in any one of Claims 1 to 9.

18. Multi-compartment device, in which a first compartment contains a dye composition as defined in any one of Claims 1 to 9 and a second compartment contains an oxidizing agent.

19. Use of the dye composition as defined in any one of Claims 1 to 12 for the oxidation dyeing of keratin fibres.

## Patentansprüche

1. Zusammensetzung zum oxidativen Färben von Keratinfasern, die in einem zum Färben geeigneten Medium enthält:
- mindestens eine Oxidationsbase, die unter 4,5-Diamino-1-(β-hydroxyethyl)-1H-pyrazol, 4,5-Diamino-1-(β-methoxyethyl)-1H-pyrazol und ihren Additionssalzen ausgewählt ist; und
- mindestens einen Kuppler, der unter 2,6-Dihydroxy-3,4-dimethylpyridin und seinen Additionssalzen ausgewählt ist.

2. Zusammensetzung nach Anspruch 1, die mindestens eine ergänzende Oxidationsbase enthält, die unter den p-Phenylendiaminen, Bisphenylalkylendiaminen, p-Aminophenolen, o-Aminophenolen, heterocyclischen Basen, die von 4,5-Diamino-1-(β-hydroxyethyl)-1H-pyrazol und 4,5-Diamino-1-(β-methoxyethyl)-1H-pyrazol verschieden sind, sowie deren Additionssalzen ausgewählt ist.

3. Zusammensetzung nach Anspruch 2, wobei die Oxidationsbasen unter den Bisphenylalkylendiaminen, p-Aminophenolen, o-Aminophenolen, heterocyclischen Basen, die von 4,5-Diamino-1-(β-hydroxyethyl)-1H-pyrazol und 4,5-Diamino-1-(β-methoxyethyl)-1H-pyrazol verschieden sind, sowie deren Additionssalzen ausgewählt sind.

4. Zusammensetzung nach Anspruch 1, die als einzige Oxidationsbase mindestens eine Oxidationsbase enthält, die unter 4,5-Diamino-1-(β-hydroxyethyl)-1H-pyrazol, 4,5-Diamino-1-(β-methoxyethyl)-1 H-pyrazol und deren Additionssalzen ausgewählt ist.

5. Zusammensetzung nach einem der vorhergehenden Ansprüche, die mindestens einen ergänzenden Kuppler enthält, der unter den m-Phenylendiaminen, m-Aminophenolen, m-Dihydroxybenzolen, Naphthalinkupplern, heterocyclischen Kupplern, die von 2,6-Dihydroxy-3,4-dimethylpyridin verschieden sind, sowie deren Additionssalzen ausgewählt ist.

6. Zusammensetzung nach einem der vorhergehenden Ansprüche, bei der der Mengenanteil jeder Oxidationsbase im Bereich von etwa 0,001 bis 10 Gew.-% des Gesamtgewichts der Farbmittelzusammensetzung liegt.

7. Zusammensetzung nach einem der vorhergehenden Ansprüche, bei der der Mengenanteil jedes Kupplers im Bereich von etwa 0,001 bis 10 Gew.-% des Gesamtgewichts der Farbmittelzusammensetzung liegt.

8. Zusammensetzung nach einem der vorhergehenden Ansprüche, die einen pH-Wert im Bereich von 3 bis 12 aufweist.

9. Zusammensetzung nach Anspruch 8, die einen pH-Wert im Bereich von 7 bis 12 aufweist.

10. Zusammensetzung nach einem der vorhergehenden Ansprüche, die ein Oxidationsmittel enthält.

11. Zusammensetzung nach Anspruch 10, bei der das Oxidationsmittel unter Wasserstoffperoxid, Harnstoffperoxid, Alkalimetallbromaten, Salzen von Persäuren, Persäuren und Oxidasen ausgewählt ist.

12. Zusammensetzung nach Anspruch 10 oder 11, die eine pH-Wert von 7 bis 12 aufweist.

13. Verfahren zum oxidativen Färben von Keratinfasern, **dadurch gekennzeichnet, dass** eine Zusammensetzung nach einem der Ansprüche 1 bis 9 auf die Keratinfasern aufgetragen und die Farbe mithilfe eines Oxidationsmittels gebildet wird.

14. Verfahren nach Anspruch 13, wobei die Farbe bei einem pH-Wert von 7 bis 12 gebildet wird.

15. Verfahren nach Anspruch 13 oder 14, wobei das Oxidationsmittel unter Wasserstoffperoxid, Harnstoffperoxid, Alkalimetallbromaten, Salzen von Persäuren, Persäuren oder Oxidasen ausgewählt ist.

16. Verfahren nach einem der Ansprüche 13 bis 15, wobei das Oxidationsmittel bei der Anwendung mit der in einem der Ansprüche 1 bis 9 definierten Zusammensetzung vermischt wird.

17. Verfahren nach einem der Ansprüche 13 bis 15, wobei das Oxidationsmittel auf die Keratinfasern in Form einer oxidierenden Zusammensetzung gleichzeitig mit oder nach der Zusammensetzung nach einem der Ansprüche 1 bis 9 aufgebracht wird.

18. Vorrichtung mit mehreren Abteilungen, wobei eine erste Abteilung eine Farbmittelzusammensetzung nach einem der Ansprüche 1 bis 9 und eine zweite Abteilung ein Oxidationsmittel enthält.

19. Verwendung der Farbmittelzusammensetzung nach einem der Ansprüche 1 bis 12 zum oxidativen Färben von Keratinfasern.
